# EUROPEAN PATENT SPECIFICATION

(11) **EP 1 789 433 B1**
(45) Date of publication and mention of the grant of the patent: **06.06.2012**
(21) Application number: 04778941.7
(22) Date of filing: 21.07.2004
(51) Int. Cl.: C07K 1/00, A61K 31/498, A61P 5/24

(54) **METHODS OF TREATING CUTANEOUS FLUSHING USING SELECTIVE ALPHA-2-ADRENERGIC RECEPTOR AGONISTS**
VERFAHREN ZUR BEHANDLUNG VON ERRÖTEN DER HAUT UNTER VERWENDUNG VON SELEKTIVEN AGONISTEN DES ALPHA-2-ADRENERGEN REZEPTORS
METHODES DE TRAITEMENT DE BOUFFEES VASOMOTRICES AVEC MANIFESTATION CUTANEE AU MOYEN D'AGONISTES SELECTIFS DES RECEPTEURS ALPHA-2-ADRENERGIQUES

(30) Priority: 23.07.2003 US 626037
(43) Date of publication of application: 30.05.2007
(60) Divisional application: 12165603.7
(73) Proprietor: Galderma Pharma S.A., 1000 Lausanne 30 Grey (CH)
(72) Inventor: SCHERER, Warren J., Naples, FL 34119 (US)
(74) Representative: Hatzmann, Martin
(86) International application number: PCT/US2004/023649
(87) International publication number: WO 2005/010025

(56) References cited:
- US-A- 4 315 033
- US-A- 4 800 209
- US-A1- 2004 156 873
- US-A1- 2004 266 776
- STEARNS VERED ET AL: "Cooling off hot flashes." JOURNAL OF CLINICAL ONCOLOGY : OFFICIAL JOURNAL OF THE AMERICAN SOCIETY OF CLINICAL ONCOLOGY 15 MAR 2002 LNKD- PUBMED:11896087, vol. 20, no. 6, 15 March 2002 (2002-03-15) , pages 1436-1438, XP002590831 ISSN: 0732-183X
- DATABASE MEDLINE [Online] US NATIONAL LIBRARY OF MEDICINE (NLM), BETHESDA, MD, US; April 1990 (1990-04), PARRA R O ET AL: "Treatment of post-orchiectomy hot flashes with transdermal administration of clonidine." XP002590832 Database accession no. NLM2313799 & THE JOURNAL OF UROLOGY APR 1990 LNKD- PUBMED:2313799, vol. 143, no. 4, April 1990 (1990-04), pages 753-754, ISSN: 0022-5347
- TULANDI T ET AL: "Effect of guanfacine, an alpha-adrenergic agonist, on menopausal flushing." MATURITAS OCT 1986 LNKD- PUBMED:3537635, vol. 8, no. 3, October 1986 (1986-10), pages 197-200, XP002590833 ISSN: 0378-5122
- LOMAX P ET AL: "Postmenopausal hot flushes and their management." PHARMACOLOGY & THERAPEUTICS 1993 FEB-MAR LNKD- PUBMED:8361997, vol. 57, no. 2-3, February 1993 (1993-02), pages 347-358, XP002590834 ISSN: 0163-7258
- WYMENGA A.N.M. AND SLEIJFER D.T.: 'Management of Hot Flushes in Breast Cancer Patients' ACTA ONCOLOGICA vol. 41, no. 3, 2002, pages 269 - 275, XP008014558

## Description

### Field of the Invention

The present invention relates to a composition comprising at least one α₂ adrenergic receptor agonist selected from the group consisting of brimonidine tartrate and a suitable carrier for use in treating, reducing, inhibiting, preventing and/or reversing cutaneous facial flushing caused by abnormal, endogenously-induced vasomotor instability associated with acne rosacea, menopause-associated hot flashes, hot flashes resulting from orchiectomy or ingestion of substances capable of inducing a cutaneous facial flushing reaction (e.g., alcohol, chocolate, spices), wherein the composition is to be administered by topical dermatological application.

### Background of the Invention

Facial flushing is a symptom observed in medical conditions associated with vasomotor instability. Cutaneous vasomotor instability is the term commonly used in the medical arts to refer to involuntary dilatation and reactivity of subcutaneous blood vessels. The mechanism of facial flushing involves involuntary dilation of subcutaneous arteries. The etiology underlying the initiation of facial flushing is unknown. There are essentially four common medical conditions addressed by the instant disclosure in which facial flushing occurs. These include: 1.) acne rosacea, 2.) postmenopausal hot flashes, 3.) patients who are status post surgical orchiectomy, and 4.) flushing secondary to ingestion of food substances.

Acne rosacea is a chronic dermatological disease of unknown cause characterized by facial flushing, erythema, recurrent papules and pustules, superficial telangiectasias (dilations of previously existing small blood vessels) and rhinophymia (hypertrophy of the nose with follicular dilation). The disorder is found mainly in fair-skinned patients between 30 and 50 years of age. Women are more commonly affected than men and acne rosacea is a common disorder. Because these clinical signs, rosacea was originally thought to resemble the acne (acne vulgaris) typically encountered in teenagers, however, rosacea is now known to represent a separate and distinct dermatological condition. It is estimated that approximately 13 million Americans have acne rosacea. Alcohol, stress, spicy foods and temperature extremes may exacerbate the condition.

Facial flushing associated with rosacea is due to vasomotor instability of unknown etiology. Therefore, treatments that stabilize the contractile state of cutaneous blood vessels would have a beneficial effect on this symptom. Improvement and stabilization of vascular tone via a vasoconstrictive mechanism is the approach taken by the instant disclosure. It appears that there are only two prior art patent methods of influencing vascular tone and reducing facial flushing. These include topical application of phytosphingosine (U.S. Published Application No. 2003/0068343) and nitric oxide synthetase inhibitors (WO 98/36730). Mechanistically, these differ greatly from the instant disclosure. Sphingosines are lipids that induce vasoconstriction in certain tissues via activity of specific cellular sphingosine receptors. Nitric oxide is a potent regulatory vasodilator that is produced by vascular endothelial cells. Inhibition of the enzyme that produces nitric oxide would therefore be expected to result in baseline vasoconstriction. However, the safety and tolerability of these compounds have not been established. Attempted treatment methods for facial flushing that have been published in the peer-reviewed medical literature have been limited to oral administration of the antihypertensive medication, clonidine (Guarrera *et al.,* 1982; Wilkin, 1983).

Clonidine is an alpha (α) adrenergic receptor agonist that crosses the blood-brain barrier and acts directly on the central nervous system. The chemical name of clonidine is N-(2,6-dichlorophenyl)-4,5-dihydro-1H-imidazol-2-amine. Clonidine has affinity for both the α₁ and α₂ subtypes of alpha adrenergic receptors. Traditionally, clonidine has been used to treat uncontrolled hypertension. Clonidine stimulates alpha adrenergic receptors in the brainstem, resulting in reduced sympathetic outflow that decreases renal vascular resistance, heart rate and blood pressure. Because clonidine acts directly on the central nervous system, its use is associated with multiple systemic side effects, such as bradycardia, heart block, hypotension, dizziness, dry mouth and depression. Some of the side effects may be life threatening.

For the purpose of patient convenience and desire to maintain adequate blood levels of the drug to control hypertension, clonidine has been administered via transdermal patch (U.S. Patent No. 4,201,211). However, this transdermal delivery system for clonidine is simply an alternate route of administration and does not alter its ability to affect the central nervous system or its mixed α₁ and α₂ adrenergic receptor kinetics. Topical clonidine has also been proposed to aid in alleviating neuropathic pain syndromes such as diabetic neuropathy and post-herpetic neuralgia (U.S. Patent No. 6,534,048). However, the mechanism of this analgesic action may be secondary to the release of endogenous enkephalin-like substances by the central nervous system (Nakamura *et al.,* 1988).

The editorial, Steams V. et al., "Cooling off hot flashes", Journal of Clinical Oncology, Vol. 20, No. 6 (March 15), 2002, pp. 1436-1438, indicates that several neuroendocrine agents such as antidopaminergic compounds and clonidine have been investigated for the treatment of hot flashes. Clonidine, orally or trans-dermally, is stated to be better tolerated than other neuroendocrine agents and is modestly active in reducing hot flashes.

Research has shown that vasoconstriction of small, distal subcutaneous resistance arteries depends entirely on α₂ adrenergic receptor stimulation (Chotani *et al.,* 2000; Nielson *et al.,* 1989). Unfortunately, because of its mixed α₁ and α₂ activity, oral dosages of clonidine sufficient to produce peripheral cutaneous vasoconstriction via α₂ adrenergic receptor stimulation would also result in intolerable systemic side effects. Hot flashes are sudden sensations of flushing and heat that some women experience when they are going through menopause. Although their etiology is not completely understood, it is thought that a decrease in the female hormone estrogen leads to vasomotor instability. Symptoms include redness and warmth of the skin of the face, neck and shoulders, pounding heartbeat and sweating. Hot flashes last from a few minutes to a half hour. Approximately 20% of women seek medical treatment for postmenopausal symptoms associated with vasomotor instability. Similar symptoms are experienced by men with prostate cancer who undergo orchiectomy (surgical removal of the testes). Treatment of hot flashes requires oral estrogen replacement therapy which is thought to raise the core body temperature sweating threshold (Freedman *et al.,* 2002). However, many patients have relative or absolute contraindications to estrogen replacement therapy (*e.g.,* history of breast cancer). These patients would benefit from a safe, locally-acting compound that alleviates facial flushing. Although the exact etiology of hot flashes is unknown, the underlying physiological mechanism of facial flushing (dilation of subcutaneous arteries) is similar to that observed with rosacea in that there is endogenously-induced vasomotor instability. Treatment of the reactive cutaneous vascular bed with the (2-imidazolin-2-ylamino) quinoxaline derivative brimonidine tartrate would stimulate α₂ adrenergic receptors, thus restoring vascular tone and reversing or preventing the cutaneous flushing reaction.

It is known that patients with vasomotor instability are also susceptible to facial flushing following the ingestion of certain foods such as alcohol, chocolate, caffeine or spices. Flushing associated with ingested substances is primarily limited to the "blush area" of the mid face (Wilkin, 1988).

Topical brimonidine tartrate eye drops have been FDA approved for the treatment of elevated intraocular pressure. In addition to treating elevated intraocular pressure, brimonidine tartrate eye drops have also been patented for treating neural injury secondary to glaucoma, retinitis pigmentosa and age related macular degeneration (U.S. Patent No. 6,194,415). Brimonidine tatrate is known to have 10 fold more α₂ adrenergic receptor activity than clonidine (Burke *et al.,* 1996) and because of its hydrophilic composition, is capable of acting locally and is unable to cross the blood-brain barrier and therefore, unable to directly influence the central nervous system (Chien et al., 1990). Toxicity studies have proven brimonidine tartrate to be nontoxic and to have no oncogenic or teratogenic activity (Walters, 1996).

### Brief Summary of the Invention

The instant invention provides a composition comprising at least one α₂ adrenergic receptor agonist selected from the group consisting of brimonidine and brimonidine tartrate for use in the treatment of cutaneous facial flushing caused by vasomotor instability as defined in claim 2 appended hereto.

One objective of the present invention involves local cutaneous application of an effective amount of at least one of the aforesaid α₂ adrenergic receptor agonists with an ability to act locally and inability to cross the blood-brain barrier to treat facial flushing reactions caused by vasomotor instability. In certain preferred embodiments, the α₂ adrenergic receptor agonist is bromonidine tartrate.

Thus, the instant disclosure describes a composition for use in a method of safely treating facial flushing in humans. The composition comprises an effective amount of at least one of the aforesaid α₂ adrenergic receptor agonists wherein the α₂ adrenergic receptor agonist is admixed with a dermatologically acceptable carrier or a pharmaceutically acceptable carrier. The compounds due to their properties as a highly specific, locally-acting α₂ adrenergic receptor agonist, act to reduce cutaneous flushing via vasoconstriction of subcutaneous arteries.

### Detailed Disclosure of the Invention

The subject invention provides a composition comprising at least one of the aforesaid selective α₂ adrenergic receptor agonists and a carrier the composition for use in methods for the treatment of facial flushing in an individual comprising the topical administration of in an amount sufficient to prevent, reduce, ameliorate, or inhibit facial flushing. In a preferred embodiment, brimonidine tartrate is an α₂ adrenergic receptor agonist used in the formulation of the compositions. In various aspects of the subject invention, the individual is a human. In other embodiments, the subject invention is for use in the treatment of flushing in individuals or humans.

Selective α₂ adrenergic receptor agonists suitable for use in the subject invention include the (2-imidazolin-2-ylamino) quinoxaline derivatives brimonidine and brimonidine tartrate. Brimonidine tartrate is a quinoxaline derivative and quinoxaline derivatives having α₂ receptor agonist activity were originally suggested as therapeutic agents by U.S. Patent No. 4,029,792.

The phrase "selective α₂ adrenergic receptor agonist(s)" is intended to convey an agonist (or agonists) that are more selective for the α₂ adrenergic receptor as compared to the α₁ adrenergic receptor. In certain embodiments of the invention, "selective α₂ adrenergic receptor agonist(s)" are at least ten (10) to 1000-fold (or higher) more selective for the α₂ adrenergic receptor than the α₁ adrenergic receptor. Brimonidine is 7-12 fold more selective for the α₂ adrenergic receptor than is clonidine

For the treatment of facial flushing in humans, one embodiment of the subject invention provides the (2-imidazolin-2-ylamino) quinoxaline derivative, brimonidine tartrate, admixed with a dermatologically acceptable carrier which is to be administered topically to skin. Any suitable, conventional, dermatologically acceptable carrier may be employed. A carrier is dermatologically acceptable if it does not inhibit the effectiveness of the active compound(s) and it has substantially no long term or permanent detrimental effect on the skin to which it is administered.

The compositions encompassed by this invention include formulations for topical application to the human skin. For topical application, one or more the aforesaid α₂ adrenergic receptor agonists, *i.e.* the (2-imidazolin-2-ylamino) quinoxaline derivatives brimonidine and brimonidine tartrate, may be formulated into any pharmaceutical form normally employed for such an application, in particular in the form of aqueous, aqueous/alcoholic or oily solutions, dispersions of lotion or serum type, aqueous anhydrous or lipophilic gels, emulsions of liquid or semi-liquid consistency of the milk type, obtained by dispersion of a fatty phase in an aqueous phase or conversely an aqueous phase in a fatty phase, or suspensions or emulsions of semi-solid or solid consistency of the cream or gel type, soaps or detergents, or alternatively microemulsions, microcapsules, microparticles, or vesicle dispersions of ionic and/or non-ionic type. Among additional alternative means for topical application of compositions according to the subject invention are spray pumps, aerosol dispersions, impregnated cosmetic facial masks, and impregnated cosmetic facial cloths or sponges. These formulations may be produced by conventional techniques.

Preparation of the compositions comprising one or more of the aforementioned α₂ adrenergic receptor agonists admixed with dermatologically accepted carriers would also include standard acids, bases and buffers including, but not limited to substances such as sodium hydroxide and lactic acid to adjust and optimize pH between approximately 6.0 and 8.5..

Compositions of the subject invention can also further comprise standard dermatological preservatives to prevent the growth of microorganisms. Such standard preservatives include substances such as benzoic acid, benzyl alcohol, phenoxyethanol and parabens. Appropriate binding agents or other substances may be included to alter the viscosity or color of the final preparation.

Compositions provided by the subject invention can also contain, in addition to the components discussed *supra,* compounds known to be beneficial to the treatment of acne rosacea in addition to one or more of the aforesaid α₂ adrenergic receptor agonists . These additional compounds include, and are not limited to, antibacterial agents, anthelmintic agents, antiangiogenesis agents, steroidal anti-inflammatory agents, non-steroidal anti-inflammatory agents, antioxidants or derivatives of retinoic acid, as well as halogens. Compositions according to the subject invention can also further comprise aloe for skin protection and/or a compound known to act as a sunscreen in addition to those components discussed herein. As would be apparent to the skilled artisan, compositions of the subject invention, can have any combination of the components discussed herein.

### References

U.S. Patent No. 4,029,792
U.S. Patent No. 6,194,415
U.S. Published Application No. 2003/0068343
U.S. Patent No. 4,201,211
U.S. Patent No. 6,534,048
WO98/36730
Guarrera, M. et al. (1982) "Flushing in rosacea: a possible mechanism" Archives of Dermatological Research 272(3-4):311-16.
Wilkin, J.K. (1983) "Effect of subdepressor clonidine on flushing reactions in rosacea. Change in malar thermal circulation index during prolonged flushing reactions" Archives of Dermatology 119(3):211-14.
Chotani, M.A. et al. (2000) "Silent alpha (2C)-adrenergic receptors enable cold-induced vasoconstriction in cutaneous arteries" American Journal of Physiology Heart Circulatory Physiology 278(4):H1075-83.
Nielson, H. et al. (1989) "Postjunctional alpha 2-adrenergic receptors mediate vasoconstriction in human subcutaneous resistance vessels" British Journal of Pharmacology 97(3):829-34.
Burke, J. et al. (1996) "Preclinical evaluation of brimonidine tartrate" Survey of Ophthalmology 41 (suppl):S9-S 18.
Walters, T.R. (1996) "Development and use of brimonidine in treating acute and chronic elevations of intraocular pressure: A review of safety, efficacy, dose response, and dosing studies" Survey of Ophthalmology 41 (suppl):S19-S26.
Chien, D.S. et al. (1990) "Corneal and conjunctival/scleral penetration ofp-aminoclonidine, AGN 190342 and clonidine in rabbit eyes" Current Eye Research 9:1051-59.
Freedman, R.R. et al. (2002) "Estrogen raises the sweating threshold in postmenopausal women with hot flashes" Fertility and Sterility 77(3):487-90.
Wilkin, J.K. (1988) "Why is flushing limited to a mostly facial cutaneous distribution?" Journal of the American Academy of Dermatology 19:309-13.
Nakamura, M. et al. (1988) "Peripheral analgesic action of clonidine: mediation by release of endogenous enkephalin-like substances" European Journal of Pharmacology 146:223-28.

## Claims

1. Use of a composition comprising at least one selective α₂ adrenergic receptor agonist selected from the group consisting of brimonidine and brimonidine tartrate admixed with a dermatologically acceptable carrier for the manufacture of a medicament for treating cutaneous flushing in humans caused by abnormal, endogenously-induced vasomotor instability,
wherein the cutaneous flushing is facial flushing and the flushing reaction is caused by acne rosacea, menopause-associated hot flashes, or by ingestion of a substance capable of inducing cutaneous facial flushing selected from the group consisting of alcohol, chocolate, spice, flavor-enhancing additives and mono-sodium glutamate, or is the result of hot flashes following orchiectomy,
whereby the composition is administered to said human via topical dermatological application in an amount effective to reduce, inhibit, reverse or prevent said cutaneous facial flushing.

2. A composition comprising:
at least one selective α₂ adrenergic receptor agonist selected from the group consisting of brimonidine and brimonidine tartrate and a dermatologically acceptable carrier for use in treating cutaneous flushing in an individual,
wherein the cutaneous flushing is facial flushing and the flushing reaction is caused by acne rosacea, menopause-associated hot flashes, or by ingestion of a substance capable of inducing cutaneous facial flushing selected from the group consisting of alcohol, chocolate, spice, flavor-enhancing additives and mono-sodium glutamate, or is the result of hot flashes following orchiectomy,
wherein the composition is to be administered to said individual via topical dermatological application in an amount sufficient to prevent, reduce, ameliorate or inhibit said facial flushing.

3. The use or composition for use according to claim 1 or claim 2, wherein the composition further comprises an agent, or combination of agents, selected from the group consisting of antibacterial agents, anthelmintic agents, antioxidant agents, steroidal anti-inflammatory agents, non-steroidal anti-inflammatory agents, antiangiogenic agents, and derivatives of retinoic acid.

4. The use or composition for use according to claim 1 or claim 2, wherein said α₂ adrenergic receptor agonist is brimonidine tartrate.

5. The use or composition for use according to claim 1, claim 2, or claim 3, wherein the composition further comprises: aloe; compounds that act as suncreens; or a combination of aloe and compounds that act as sunscreens.

6. The use or composition for use according to claim 1, claim 2, or claim 3, wherein the composition further comprises a preservative.

7. The use or composition for use according to claim 1, claim 2, or claim 3, wherein the composition further comprises a halogen.

## Patentansprüche

1. Verwendung einer Zusammensetzung umfassend mindestens einen selektiven α2 adrenergen Rezeptorantagonisten ausgewählt aus der Gruppe bestehend aus Brimonidin und Brimonidintartrat, vermengt mit einem dermatologisch akzeptablen Träger, zur Herstellung eines Medikaments zur Behandlung von Hautrötungen bei Menschen, die durch abnormale, endogen induzierte vasomotorische Instabilität verursacht wird,
wobei die Hautrötung eine Gesichtsrötung ist und die Rötungsreaktion durch Akne Rosacea, menopausenbedingte Hitzewallungen, oder durch die Einnahme einer Substanz, die fähig ist, Hautrötung im Gesicht zu induzieren, ausgewählt aus der Gruppe bestehend aus Alkohol, Schokolade, Gewürz, Geschmacksverstärker und Mononatriumglutamat, oder welche das Resultat von Hitzewallungen ist, die einer Hodenentfernung folgen,
wobei die Zusammensetzung dem Menschen als topisch dermatologische Applikation in einer Menge verabreicht wird, die wirksam ist, Hautröte im Gesicht zu reduzieren, zu hemmen, rückgängig zu machen oder vorzubeugen.

2. Zusammensetzung umfassend:
mindestens einen selektiven α₂ adrenergen Rezeptorantagonisten ausgewählt aus der Gruppe bestehend aus Brimonidin und Brimonidintartrat und einem dermatologisch akzeptablen Träger zur Verwendung in der Behandlung von Hautrötung in einem Individuum,
wobei die Hautrötung eine Gesichtsrötung ist, und die Rötungsreaktion durch Akne Rosazea, menopausenbedingte Hitzewallungen, oder durch die Einnahme einer Substanz, die fähig ist, Hautrötung im Gesicht zu induzieren, ausgewählt aus der Gruppe bestehend aus Alkohol, Schokolade, Gewürz, Geschmacksverstärker und Mononatriumglutamat verursacht wird, oder welche das Resultat von Hitzewallungen ist, die einer Hodenentfernung folgen,
wobei die Zusammensetzung dem Individuum als topisch dermatologisch Applikation in einer Menge zu verabreichen ist, die ausreicht, Hautröte im Gesicht vorzubeugen, zu reduzieren, zu verbessern oder zu hemmen.

3. Verwendung oder Zusammensetzung zur Verwendung nach Anspruch 1 oder Anspruch 2, wobei die Zusammensetzung ferner ein Agens oder eine Kombination von Agentien umfasst, ausgewählt aus der Gruppe bestehend aus antibakteriellen Agentien, anthelmintischen Agentien, antioxidativen Agentien, steroidalen entzündungshemmenden Agentien, nichtsteroidalen entzündungshemmenden Agentien, angiogenesehemmenden Agentien, und Derivaten der Retinolsäure.

4. Verwendung oder Zusammensetzung zur Verwendung nach Anspruch 1 oder Anspruch 2, wobei der α₂ adrenerge Rezeptorantagonist Brimonidintartrat ist.

5. Verwendung oder Zusammensetzung zur Verwendung nach Anspruch 1, Anspruch 2 oder Anspruch 3, wobei die Zusammensetzung weiter umfasst:
Aloe; Komponenten, die als Sonnenschutzmittel fungieren; oder eine Kombination von Aloe und Komponenten, die als Sonnenschutzmittel fungieren.

6. Verwendung oder Zusammensetzung zur Verwendung nach Anspruch 1, Anspruch 2 oder Anspruch 3, wobei die Zusammensetzung weiter einen Konservierungsstoff umfasst.

7. Verwendung oder Zusammensetzung zur Verwendung nach Anspruch 1, Anspruch 2 oder Anspruch 3, wobei die Zusammensetzung weiter ein Halogen umfasst.

## Revendications

1. Utilisation d'une composition comprenant au moins un agoniste sélectif des récepteurs α2-adrénergiques sélectionné dans le groupe consistant en la brimonidine et le tartrate de brimonidine mélangé(e) avec un véhicule dermatologiquement acceptable, pour la fabrication d'un médicament destiné au traitement de bouffées vasomotrices avec manifestation cutanée chez les humains provoquées par une instabilité vasomotrice anormale induite de manière endogène,
où les bouffées vasomotrices avec manifestation cutanée sont des bouffées vasomotrices du visage et la réaction de type bouffées vasomotrices est provoquée par l'acné rosacée, des bouffées de chaleur associées à la ménopause, ou par l'ingestion d'une substance capable d'induire des bouffées vasomotrices du visage avec manifestation cutanée sélectionnée dans le groupe consistant en l'alcool, le chocolat, les épices, des additifs exhausteurs de goût et le glutamate monosodique, ou est le résultat de bouffées de chaleur suite à une orchidectomie,
moyennant quoi la composition est administrée audit humain via une application dermatologique topique en une quantité efficace pour réduire, inhiber, inverser ou prévenir lesdites bouffées vasomotrices du visage avec manifestation cutanée.

2. Composition comprenant :
au moins un agoniste sélectif des récepteurs α2-adrénergiques sélectionné dans le groupe consistant en la brimonidine et le tartrate de brimonidine et un véhicule dermatologiquement acceptable pour une utilisation dans le traitement de bouffées vasomotrices avec manifestation cutanée chez un individu,
où les bouffées vasomotrices avec manifestation cutanée sont des bouffées vasomotrices du visage et la réaction de type bouffées vasomotrices est provoquée par l'acné rosacée, des bouffées de chaleur associées à la ménopause, ou par l'ingestion d'une substance capable d'induire des bouffées vasomotrices du visage avec manifestation cutanée sélectionnée dans le groupe consistant en l'alcool, le chocolat, les épices, des additifs exhausteurs de goût et le glutamate monosodique, ou est le résultat de bouffées de chaleur suite à une orchidectomie,
où la composition doit être administrée audit individu via une application dermatologique topique en une quantité suffisante pour prévenir, réduire, améliorer ou inhiber lesdites bouffées vasomotrices du visage.

3. Utilisation ou composition destinée à être utilisée selon la revendication 1 ou la revendication 2, où la composition comprend en outre un agent, ou une combinaison d'agents, sélectionné(e) dans le groupe consistant en des agents antibactériens, des agents anthelminthiques, des agents antioxydants, des agents anti-inflammatoires stéroïdiens, des agents anti-inflammatoires non stéroïdiens, des agents anti-angiogéniques, et des dérivés d'acide rétinoïque.

4. Utilisation ou composition destinée à être utilisée selon la revendication 1 ou la revendication 2, où ledit agoniste des récepteurs α2-adrénergiques est le tartrate de brimonidine.

5. Utilisation ou composition destinée à être utilisée selon la revendication 1, la revendication 2, ou la revendication 3, où la composition comprend en outre : de l'aloés ; des composés qui agissent comme des écrans solaires ; ou une combinaison d'aloès et de composés qui agissent comme des écrans solaires.

6. Utilisation ou composition destinée à être utilisée selon la revendication 1, la revendication 2, ou la revendication 3, où la composition comprend en outre un conservateur.

7. Utilisation ou composition destinée à être utilisée selon la revendication 1, la revendication 2, ou la revendication 3, où la composition comprend en outre un halogène.
